# EUROPÄISCHE PATENTSCHRIFT

(11) **EP 0 674 878 B1**
(45) Veröffentlichungstag und Bekanntmachung des Hinweises auf die Patenterteilung: **11.11.1998**
(21) Anmeldenummer: 95103222.6
(22) Anmeldetag: 07.03.1995
(51) Int. Cl.: A61B 17/28, A61B 17/32

(54) **Medizinisches Instrument**
Medical instrument
Instrument médical

(30) Priorität: 30.03.1994 DE 4411099
(43) Veröffentlichungstag der Anmeldung: 04.10.1995
(73) Patentinhaber: Richard Wolf GmbH, 75438 Knittlingen (DE)
(72) Erfinder: Heimberger, Rudolf, D-75038 Oberderdingen (DE); Schaumann, Uwe, D-75438 Knittlingen (DE)
(74) Vertreter: Wilcken, Thomas, Dipl.-Ing.

(56) Entgegenhaltungen:
- WO-A-92/05828
- WO-A-93/25267
- DE-C- 4 313 903
- US-A- 5 044 947
- US-A- 5 242 458

## Beschreibung

Die Erfindung betrifft ein medizinisches Instrument, insbesondere Zange, Schere oder Halter, mit einem distalen, aus zwei Maulteilen bestehenden Maul und einer proximalen Handhabe, bei deren Betätigung wenigstens ein Maulteil relativ zum anderen verstellbar ist, und mit einer Einrichtung zur Einstellung einer definierten maximalen Schließkraft des Maules.

Derartige medizinische Instrumente sind in vielen Ausführungsformen bekannt. So ist beispielsweise aus DE-A 36 01 166 eine Zange zum Fassen von Gewebe zu entnehmen, bei der ein festes und ein schwenkbewegliches Maulteil einem festen bzw. einem schwenkbaren Griffteil zugeordnet sind, wobei zwecks Begrenzung der an den Maulteilen erzeugbaren Druckkraft der letztgenannte Griffteil in zwei gelenkig miteinander verbundene Teilstücke aufgetrennt ist, die gegeneinander mittels eines Federgliedes abgestützt sind.

Bei Überschreiten eines vorbestimmten Druckes wird zwar das untere Teilstück gegenüber dem anderen, dann nicht mehr verschwenkbaren Teilstück ausgelenkt, beim weiteren Bewegen der Grifftelle aufeinander zu nimmt jedoch die Haltekraft der Maulteile entsprechend der Kennlinie des Federgliedes weiter zu. Die sich so erhöhende Kraft kann letztlich zur Perforation oder gar zur Durchtrennung des zwischen den Maulteilen befindlichen Gewebes führen.

Aus der DE-A 40 10 775 ist weiter eine chirurgische Zange bekannt, die ein festes Maulteil mit einem diesem zugeordneten festen Griffteil und ein bewegliches Maulteil aufweist, das mittels eines schwenkbaren Grifteiles betätigbar ist. Dabei besteht der schwenkbare Griffteil aus zwei miteinander fluchtenden Teilen, die durch ein Biegefederelement unlösbar miteinander verbunden sind. Zur Fixierung der Griffstellung der Maulteile sind an den Griffteilen Rastelemente vorgesehen, die miteinander in lösbaren Eingriff treten.

Auch bei diesem Instrument ist eine als maximal möglich vorgegebene Haltekraft mit den Maulteilen nicht erzielbar, so daß auch hier insbesondere beim Einsatz solcher Instrumente als Halter für Gefäße, Gewebe, Organe u.s.w. bei Operationen die Gefahr einer Schädigung durch übermäßige Klemmkraft der Maulteile besteht. Außerdem besteht die Gefahr, daß Teile des Instrumentes für den Fall einer übermäßigen Krafteinwirkung brechen.

Es ist die Aufgabe der Erfindung, ein Instrument der eingangs beschriebenen Art derart weiterzubilden, daß die von den Maulteilen ausgeübte Kraft nicht nur einen maximalen Wert nicht überschreiten kann, sondern auch bei weitergehendem Schließen der Handhabe nach Erfassen eines Objektes konstant bleibt.

Diese Aufgabe wird erfindungsgemäß dadurch gelöst, daß auf dem Weg der Kraftübertragung von der Handhabe zum Maul zumindest ein Kraftübertragungselement aus superelastischem, reversibel verformbarem Werkstoff mit mechanischem Formgedächtnis vorgesehen ist, dessen Spannungs-Dehnungs-Diagramm bei Spannungsbeaufschlagung ein den Bereich der Superelastizität darstellendes Plateau durchläuft, daß die Plateauspannung auf die erwähnte maximale Schließkraft abgestimmt ist und daß der Weg der Verstellung der Handhabe zum Schließen des Maules durch einen Anschlag derart begrenzt ist, daß das Kraftübertragungselement maximal mit der Plateauspannung belastbar ist.

Die Verwendung eines Kraftübertragungselementes aus einer Formgedächtnislegierung zur Übertragung der an der Handhabe erzeugten Betätigungskraft auf das Instrumentenmaul ermöglicht die Erzeugung einer Schließkraft, die nach Erfassen eines Objektes und nach Erreichen der als maximal vorgegebenen Schließkraft selbst bei weitergehender Betätigung der Handhabe in Schließrichtung konstant bleibt. Bei diesen Formgedächtnislegierungen wird die Eigenschaft der Super- oder Pseudoelastizität genutzt, die bei in bestimmter Weise wärmebehandelten Bauteilen aus z. B. Ni-Ti-Legierungen erreichbar ist. Diese Eigenschaft äußert sich bei einer bestimmten Belastung in einem Verhalten, das dem des Gummis ähnlich ist, d. h., es sind bei solchen Bauteilen relativ hohe Verformungswege ohne wesentlichen Anstieg der Verformungskraft möglich. Bei Belastungen innerhalb dieses Bereiches erfolgt selbst nach einer Dehnung bis zu 8% bei Entlastung eine Rückformung in die ursprüngliche Gestalt. Diese Eigenschaft des Formgedächtnisses wird bei dem erfindungsgemäßen Instrument gezielt genutzt, indem durch geeignete Dimensionierung des Kraftübertragungselementes und Begrenzung des Betätigungsweges der Handhabe dafür Sorge getragen wird, daß sich das Kraftübertragungselement maximal mit einer Spannung belasten läßt, die die erwähnte Plateauspannung nicht übersteigt und die auch nur so hoch ist, daß das Instrument nicht zu Bruch gehen kann.

Dieser Effekt kann in einfacher Weise bei einem Instrument verwirklicht werden, bei dem die Maulteile an dem distalen Ende und die Handhabe an dem proximalen Ende eines Schaftrohres angeordnet sind, wobei die Handhabe einen mit dem Schaftrohr starr verbundenen Griffteil und einen diesem zugeordneten beweglichen Griffteil umfaßt, und zwar dadurch, daß
a. das Kraftübertragungselement als Zug-Druckstange ausgebildet ist, die in dem Schaftrohr geführt ist und die mindestens eines der Maulteile mit dem beweglichen Griffteil in dem Sinne verbindet, daß das Kraftübertragungselement beim Schließen der Maulteile mit einer Zugkraft belastet wird, die höchstens der Plateauspannung des Formgedächtnismateriales entspricht, oder
b. das Kraftübertragungselement als Biegeelement ausgebildet ist, das zwischen dem beweglichen Griffteil der Handhabe und dem proximalen Ende einer in dem Schaftrohr geführten und den beweglichen Griffteil mit dem Instrumentenmaul verbindenden Betätigungsstange angeordnet ist, oder
c. das Kraftübertragungselement als in dem Schaftrohr geführte Zugfeder ausgebildet ist, deren proximales Ende mit dem beweglichen Griffteil und deren distales Ende mit wenigstens einem der Maulteile in Verbindung steht, oder
d. das Kraftübertragungselement als in dem Schaftrohr geführte Druckfeder ausgebildet ist, deren distales Ende mit dem beweglichen Griffteil und deren proximales Ende mit dem Instrumentenmaul Verbindung hat.

Schließlich kann auch ein Instrument, welches scherenartig zu schließen und zu öffnen ist und ein Paar Griffelemente umfaßt, deren jedes proximalseitig mit einer Fingeraufnahme und distalseitig mit einem Paar Maulteile versehen ist und bei dem beide Griffteile ein gemeinsames Schwenklager aufweisen, erfindungsgemäß modifiziert werden dadurch, daß als Kraftübertragungselement ein Zwischenstück aus einer Formgedächtnislegierung vorgesehen ist, das einen integralen Bestandteil mindestens eines der Griffelemente bildet.

Die Erfindung wird nachstehend anhand von in der Zeichnung dargestellten Ausführungsbeispielen näher erläutert. Es zeigen
- Fig. 1: ein medizinisches Instrument als Greifzange in Seitenansicht in deaktiviertem Zustand,
- Fig. 2: das Instrument nach Fig. 1 in aktiviertem Zustand,
- Fig. 3: ein Instrument entsprechend Fig. 1 mit einem modifiziertem Übertragungselement in aktiviertem Zustand,
- Fig. 4: das Instrument nach Fig. 3 in deaktiviertem Zustand,
- Fig. 5: eine weitere Ausführung des Instrumentes entsprechend Fig. 1 mit einem modifizierten Übertragungselement in aktiviertem Zustand,
- Fig. 6: einen Teil des Instrumentes nach Fig. 5 in deaktiviertem Zustand,
- Fig. 7: eine weitere Ausführung des Instrumentes entsprechend Fig. 1 mit einem modifiziertem Übertragungselement in aktiviertem Zustand,
- Fig. 8: einen Teil des Instrumentes nach Fig. 7 in deaktiviertem Zustand,
- Fig. 9: ein scherenförmig gestaltetes medizinisches Instrument und
- Fig. 10: ein die Spannungs-Dehnungs-Charakteristik einer Formgedächtnislegierung darstellendes Diagramm.

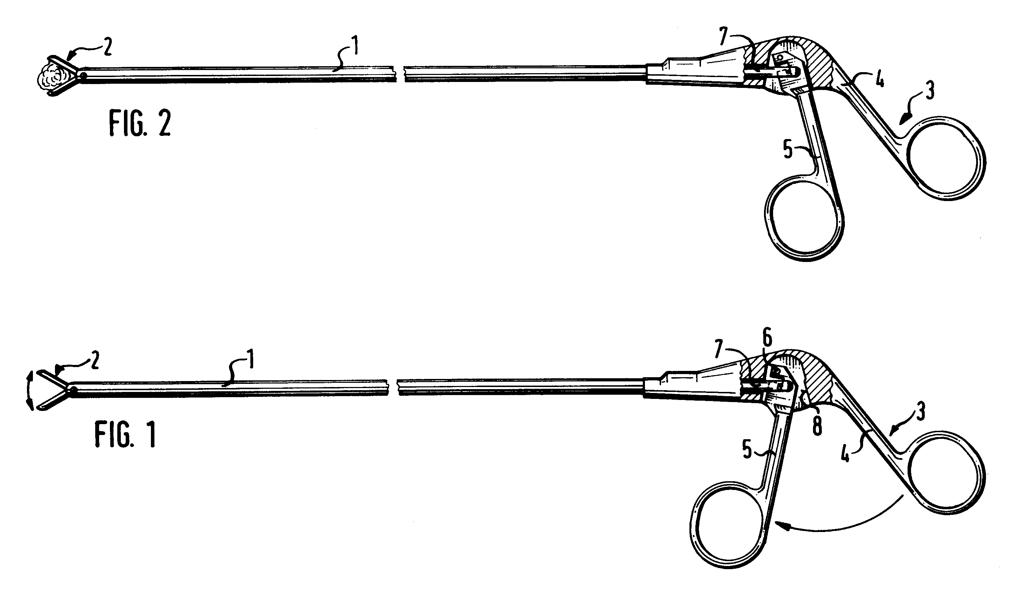

Das Instrument nach Fig. 1 besteht im wesentlichen aus einem Schaftrohr 1, einem Paar Maulteile 2, die als Scheren-, Zangen- oder Faßmaulteile ausgebildet sein können und einer Handhabe 3.

Die Maulteile 2 sind an dem distalen Ende des Schaftrohres 1 schwenkbar gelagert, und die Handhabe 3 ist an dem proximalen Ende des Schaftrohres 1 angeordnet. Die Handhabe 3 besteht aus einem mit dem Schaftrohr 1 starr verbundenen Griffteil 4 und einem an diesem um einen Lagerstift 6 schwenkbar gelagerten beweglichen Griffteil 5. In dem Schaftrohr 1 ist eine Zug-Druckstange 7 geführt, die proximalseitig mit dem beweglichen Griffteil 5 und distalseitig mit den Maulteilen 2 derart gekoppelt ist, daß eine axiale Bewegung der Zug-Druckstange 7 in bekannter Weise in eine Schwenkbewegung der Maulteile 2 umgesetzt wird. Dies wird durch Betätigen des beweglichen Griffteiles 5 bewirkt und veranlaßt bei Schwenken dieses Griffteiles 5 in Richtung auf den starren Griffteil 4 eine Schließbewegung der Maulteile 2.

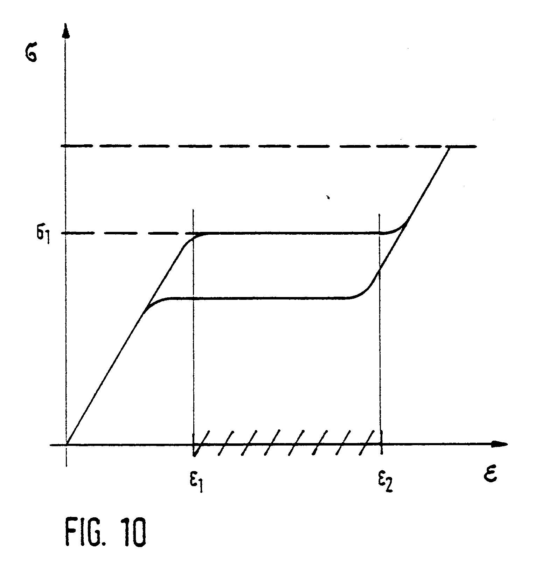

Die Zug-Druckstange 7 ist aus einem Werkstoff mit mechanischem Formgedächtnis gefertigt, dessen Spannungs-Dehnungsverhalten in Fig. 10 dargestellt ist. Erreichen die Maulteile ihre Schließstellung oder treffen sie auf einen Widerstand, beispielsweise ein Gewebestück, wie in Fig. 2 angedeutet, so wächst zunächst die Haltekraft an den Maulteilen 2 entsprechend der Betätigungskraft an der Handhabe 3, bis sie einen Wert σ 1 (Plateauspannung) erreicht. Wird nun der bewegliche Griffteil 5 weiterbewegt, so erfährt die Zug-Druckstange 7 eine Dehnung, die sich innerhalb des Dehnungsintervalles ε 1 - ε 2 unter gleichbleibender Kraft zwischen den Maulteilen 2 vollzieht. Dies bedeutet, daß die in der Zug-Druckstange 7 wirksame Spannung im Bereich dieses Dehnungsintervalles auf den Wert σ 1 begrenzt bleibt. Um zu vermeiden, daß der Dehnungsgrenzwert ε 2, an dem die maximal mögliche Schließkraft des Zangenmaules und auch die Kraft orientiert sind, mit der das Instrument zur Vermeidung einer Bruchgefahr höchstens belastet werden darf, überschritten wird und eine irreversible plastische Verformung der Stange 7 auftritt, ist ein Anschlag 8 vorgesehen, der den beweglichen Griffteil 5 nach einem vorgegebenen Schwenkweg abfängt. Bei Entlastung des Griffteiles 5 bildet sich die Dehnung der Zug-Druckstange 7 elastisch zurück, wobei der Spannungsverlauf dem unteren Linienzug des Diagrammes nach Fig. 10 folgt.

Durch diesen Aufbau kann ausgeschlossen werden, daß das gefaßte Objekt beispielsweise zerdrückt wird. Die Zug-Druckstange 7 wird also so dimensioniert, daß z. B. beim Erfassen harter Gewebeteile oder Knorpel trotz weiterer Betätigung der Handhabe in Schließrichtung, d. h. beim weiteren Zusammendrücken der Griffteile, die auf die Maulteile 2 ausgeübte Kraft konstant bleibt, so daß auch eine das Instrument schädigende Überlastung vermieden wird.

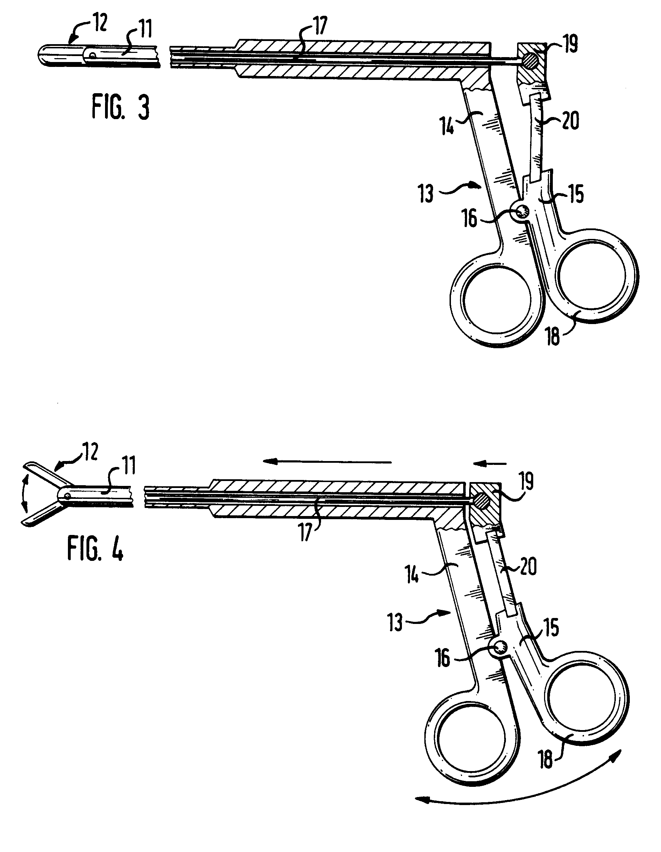

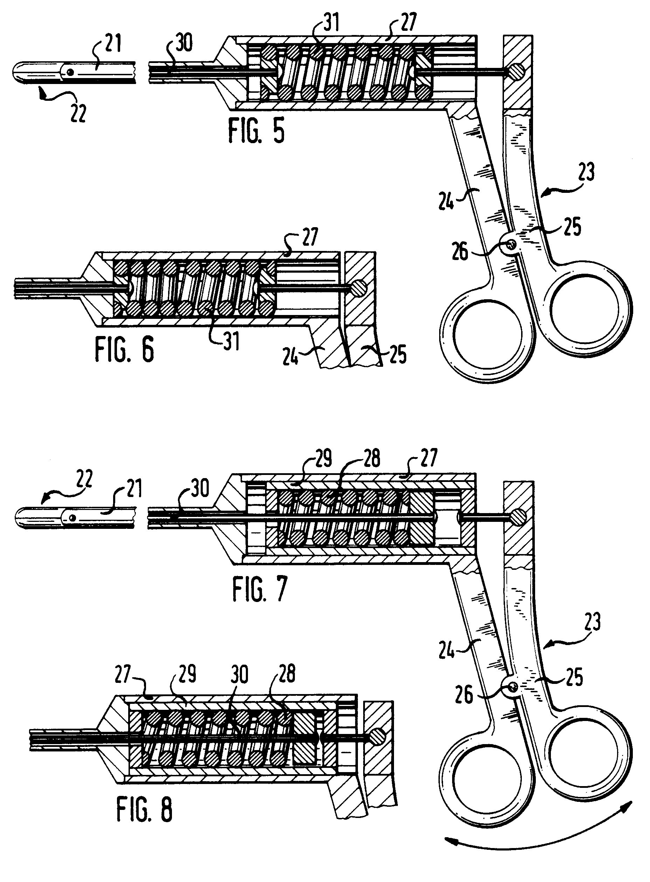

Das in den Figuren 3 und 4 gezeigte Instrument weist wie das nach Fig. 1 ein Schaftrohr 11 auf, das an seinem distalen Ende mit einem Paar schwenkbarer Maulteile 12 und an seinem proximalen Ende mit dem starren Griffteil 14 einer Handhabe 13 verbunden ist. Am Griffteil 14 ist ein beweglicher Griffteil 15 um einen Lagerstift 16 schwenkbar gelagert. Im Schaftrohr 11 ist eine Betätigungsstange 17 geführt, die entsprechend der Ausführung nach Fig. 1 distalseitig mit den Maulteilen 12 und proximalseitig mit dem beweglichen Griffteil 15 gekoppelt ist. Der Griffteil 15 besteht aus einem mit einer Fingeraufnahme versehenen Betätigungsteil 18 und einem Aufnahmeteil 19 für die Betätigungsstange 17, und die Teile 18 und 19 sind durch ein Zwischenstuck 20 aus einer Legierung mit Formgedächtniseigenschaften verbunden. Der Schwenkweg des Griffteiles 15 ist begrenzt.

Wie aus Fig. 3 ersichtlich, verformt sich das Zwischenstück 20 bei Überschreiten einer vorgegebenen Schließkraft an den Maulteilen 12, wobei die gewählte Ausführung des Zwischenstückes 20 bewirkt, daß diese Verformung nach dem Diagramm entsprechend Fig. 10 bei einer Spannung σ 1 einsetzt und innerhalb der Dehnungsgrenzen ε 1 und ε 2 vor sich geht und spätestens bei Erreichen des Wertes ε 2 endet, was durch einen Anschlag sichergestellt wird, der in diesem Fall durch die Griffteile 14, 15 gebildet wird, indem diese gemäß Fig. 3 gegenseitig zur Anlage gelangen. Auch bei dieser Ausführung des Instrumentes wird die Verformungsspannung σ 1 so festgelegt, daß eine Überbeanspruchung des erfaßten Objektes und/oder des Instrumentes ausgeschlossen ist.

Die in den Figuren 5 bis 8 gezeigten Instrumente weisen ein Schaftrohr 21 auf, das an seinem distalen Ende mit einem Paar schwenkbarer Maulteile 22 und an seinem proximalen Ende mit dem starren Griffteil 24 einer Handhabe 23 versehen ist. An dem Griffteil 24 ist ein beweglicher Griffteil 25 um einen Lagerstift 26 schwenkbar gelagert. Das Schaftrohr 21 ist in seinem proximalen Endbereich mit einem erweiterten rohrförmigen Teil 27 ausgeführt, der nach Figuren 7 und 8 der Aufnahme einer Druckfeder 28 aus einer Legierung mit Formgedächtniseigenschaften dient. Die Druckfeder 28 ist in einem in dem Teil 27 längsbewegbaren Rohrteil 29 untergebracht, das die Druckfeder 28 an ihrem distalen Ende hinterfaßt und proximalseitig mit dem beweglichen Griffteil 25 gekoppelt ist. Das distale Ende der Druckfeder 28 ist mit einer Zug-Druckstange 30 verbunden, die distalseitig mit den Maulteilen 22 gekoppelt ist.

Bei Betätigung des Griffteiles 25 entsprechend Fig. 7 erfolgt der Kraftfluß zu den Maulteilen 22 über die Druckfeder 28, deren Verformungsverhalten wiederum wie vorher beschrieben so genutzt wird, daß eine Überbeanspruchung von erfaßten Objekten und/oder des Instrumentes ausgeschlossen ist.

Die Ausführung nach Figuren 5 und 6 hat ein als Zugfeder 31 ausgebildetes Übertragungselement. Dabei ist die Zugfeder 31 direkt in dem erweiterten Teil 27 des Schaftrohres 21 geführt und distalseitig mit der mit den Maulteilen 22 gekoppelten Zug-Druckstange 30 und proximalseitig mit dem beweglichen Griffteil 25 verbunden. Die Betätigung des Griffteiles 25 in Richtung auf den Griffteil 24 bewirkt einen Kraftfluß, der als Zugspannung über die Zugfeder 31 verläuft, wobei wiederum der vorstehend beschriebene Effekt erreicht wird.

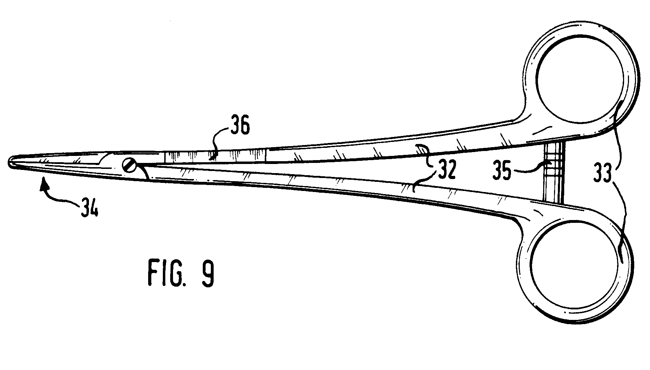

Die Ausführung nach Fig. 9 zeigt ein scherenförmig ausgestaltetes Instrument mit einem Paar gelenkig miteinander verbundenen Griffelementen 32, deren jedes proximalseitig mit einer Fingeraufnahme 33 und distalseitig mit einem Maulteil 34 versehen ist. Zwischen den beiden Griffelementen 32 ist eine Rastsicherung 35 angeordnet, die eine Fixierung der jeweiligen Stellung der Griffelemente 32 ermöglicht.

Auch bei derartigen z. B. als Nadelhalter dienenden Instrumenten wird oft eine maximale konstante Haltekraft benötigt, bzw. ist ein Schutz des Instrumentes vor Überlastung erforderlich. Dieser Schutz ist besonders wichtig, weil abgebrochene Teile des Instrumentes während des Einsatzes in einer Körperhöhle in diese fallen können und möglicherweise nur noch operativ entfernt werden können. Diese Probleme lassen sich bei solchen Instrumenten auf einfache Weise dadurch lösen, daß mindestens eines der beiden Griffelemente 32 ein Kraftübertragungselement 36 aufweist, das integraler Bestandteil des betreffenden Griffelementes 32 ist und aus einer Legierung mit Formgedächtniseigenschaften besteht.

## Patentansprüche

1. Medizinisches Instrument, insbesondere Zange, Schere oder Halter, mit einem distalen, aus zwei Maulteilen (2, 12, 22, 34) bestehenden Maul und einer proximalen Handhabe (3, 13, 23, 32), bei deren Betätigung wenigstens ein Maulteil relativ zum anderen verstellbar ist, und mit einer Einrichtung zur Einstellung einer definierten maximalen Schließkraft des Maules, dadurch gekennzeichnet, daß auf dem Weg der Kraftübertragung von der Handhabe (3, 13, 23, 32) zum Maul zumindest ein Kraftübertragungselement (7, 20, 28, 31, 36) aus superelastischem, reversibel verformbaren Werkstoff mit mechanischem Formgedächtnis vorgesehen ist, dessen Spannungs-Dehnungs-Diagramm bei Spannungsbeaufschlagung ein den Bereich der Superelastizität darstellendes Plateau durchläuft, daß die Plateauspannung (σ 1) auf die erwähnte maximale Schließkraft abgestimmt ist und daß der Weg der Verstellung der Handhabe zum Schließen des Maules durch einen Anschlag (8) derart begrenzt ist, daß das Kraftübertragungselement (7, 20, 28, 31, 36) maximal mit der Plateauspannung belastbar ist.

2. Medizinisches Instrument nach Anspruch 1, bei dem die Maulteile (2, 12, 22) an dem distalen Ende und die Handhabe (3, 13, 23) an dem proximalen Ende eines Schaftrohres (1, 11, 21) angeordnet sind, wobei die Handhabe einen mit dem Schaftrohr starr verbundenen Griffteil (4, 14, 24) und einen diesem zugeordneten beweglichen Griffteil (5, 15, 25) umfaßt, dadurch gekennzeichnet, daß das Kraftübertragungselement als Zug-Druckstange (7) ausgebildet ist, die in dem Schaftrohr (1) geführt ist und die die Maulteile (2) mit dem beweglichen Griffteil (5) in dem Sinne verbindet, daß das Kraftübertragungselement (7) beim Schließen der Maulteile mit einer Zugkraft belastet wird.

3. Medizinisches Instrument nach Anspruch 1, bei dem die Maulteile (2, 12, 22) an dem distalen Ende und die Handhabe (3, 13,, 23) an dem proximalen Ende eines Schaftrohres (1, 11, 21) angeordnet sind, wobei die Handhabe einen mit dem Schaftrohr starr verbundenen Griffteil (4, 14, 24) und einen diesem zugeordneten beweglichen Griffteil (5, 15, 25) umfaßt, dadurch gekennzeichnet, daß das Kraftübertragungselement als Biegeelement (20) ausgebildet ist, das zwischen dem beweglichen Griffteil (15) und dem proximalen Ende einer in dem Schaftrohr (11) geführten und den beweglichen Griffteil (15) mit den Maulteilen (12) verbindenden Betätigungsstange (17) angeordnet ist.

4. Medizinisches Instrument nach Anspruch 1, bei dem die Maulteile (2, 12, 22) an dem distalen Ende und die Handhabe (3, 13,, 23) an dem proximalen Ende eines Schaftrohres (1, 11, 21) angeordnet sind, wobei die Handhabe einen mit dem Schaftrohr starr verbundenen Griffteil (4, 14, 24) und einen diesem zugeordneten beweglichen Griffteil (5, 15, 25) umfaßt, dadurch gekennzeichnet, daß das Kraftübertragungselement als in dem Schaftrohr (21) geführte Zugfeder (31) ausgebildet ist, deren proximales Ende mit dem beweglichen Griffteil (25) und deren distales Ende mit den Maulteilen (22) Verbindung hat.

5. Medizinisches Instrument nach Anspruch 1, bei dem die Maulteile (2, 12, 22) an dem distalen Ende und die Handhabe (3, 13,, 23) an dem proximalen Ende eines Schaftrohres (1, 11, 21) angeordnet sind, wobei die Handhabe einen mit dem Schaftrohr starr verbundenen Griffteil (4, 14, 24) und einen diesem zugeordneten beweglichen Griffteil (5, 15, 25) umfaßt, dadurch gekennzeichnet, daß das Kraftübertragungselement als eine in dem Schaftrohr (21) geführte Druckfeder (28) ausgebildet ist, deren proximales Ende mit dem beweglichen Griffteil (25) und deren distales Ende mit den Maulteilen (22) Verbindung hat.

6. Medizinisches Instrument nach Anspruch 1 mit einem scherenartigen Aufbau und einem Paar Griffelemente (32), die proximalseitig mit einer Fingeraufnahme (33) und distalseitig mit einem Paar Maulteile (34) versehen ist und bei dem beide Griffelemente (32) ein gemeinsames Schwenklager aufweisen, dadurch gekennzeichnet, daß als Kraftübertragungselement ein Zwischenstück (36) aus einer Formgedächtnislegierung vorgesehen ist, das einen integralen Bestandteil mindestens eines der Griffelemente (32) bildet.

## Claims

1. A medical instrument, in particular a forceps, scissors or holder, with a distal jaw consisting of two jaw parts (2, 12, 22, 34) and with a proximal handle (3, 13, 23, 32) on whose actuation at least one jaw part can be adjusted relative to the other, and with a means for setting a defined maximum closing force of the jaw, characterised in that on the path of force transmission from the handle (3, 13, 23, 32) to the jaw at least one force transmission element (7, 20, 28, 31, 36) of super-elastic reversibly deformable material with a shape-memory is provided whose stress-extension diagram with a loading with tension runs through a plateau representing a region of super-elasticity, that the plateau stress (σ 1) is matched to the mentioned maximum closing force and that the path of the adjustment of the handle for closing the jaw is limited by an abutment (8) in a manner such that the force transmission element (7, 20, 28, 31, 36) is maximumly loadable with the plateau stress.

2. A medical instrument according to claim 1, with which the jaw parts (2, 12, 22) are arranged at the distal end of a shank tube (1, 11, 21) and the handle (3, 13, 23) at the proximal end, wherein the handle comprises a grip part (4, 14, 24) rigidly connected to the shank tube and a movable grip part (5, 15, 25) allocated to this, characterised in that the force transmission element is formed as a pull-push rod (7) which is guided in the shank tube (1) and which connects the jaw parts (2) with the movable grip part (5) in the sense that the force transmission element (7) on closing the jaw parts is loaded with a tension force.

3. A medical instrument according to claim 1, with which the jaw parts (2, 12, 22) are arranged at the distal end of a shank tube (1, 11, 21) and the handle (3, 13, 23) at the proximal end, wherein the handle comprises a grip part (4, 14, 24) rigidly connected to the shank tube and a movable grip part (5, 15, 25) allocated to this, characterised in that the force transmission element is formed as a bending element (20) which is arranged between the movable grip part (15) and the proximal end of an operating rod (17) which is guided in the shank tube (11) and connects the movable grip part (15) with the jaw parts (12).

4. A medical instrument according to claim 1, with which the jaw parts (2, 12, 22) are arranged at the distal end of a shank tube (1, 11, 21) and the handle (3, 13, 23) at the proximal end, wherein the handle comprises a grip part (4, 14, 24) rigidly connected to the shank tube and a movable grip part (5, 15, 25) allocated to this, characterised in that the force transmission element is formed as a tension spring (31) guided in the shank tube (21), whose proximal end has a connection to the movable grip part (25) and whose distal end a connection to the jaw parts (22).

5. A medical instrument according to claim 1, with which the jaw parts (2, 12, 22) are arranged at the distal end of a shank tube (1, 11, 21) and the handle (3, 13, 23) at the proximal end, wherein the handle comprises a grip part (4, 14, 24) rigidly connected to the shank tube and a movable grip part (5, 15, 25) allocated to this, characterised in that the force transmission element is formed as a compression spring (28) guided in the shank tube (21), whose proximal end has a connection to the movable grip part (25) and whose distal end a connection to the jaw parts (22).

6. A medical instrument according to claim 1 with a scissor-like construction and a pair of grip elements (32) which on the proximal side are provided with a finger receiver (33) and on the distal side with a pair of jaw parts and with which both grip elements (32) comprise a common pivoting bearing, characterised in that as a force transmission element an intermediate piece (36) of a memory shape alloy is provided which forms an integral component of at least one of the grip elements (32).

## Revendications

1. Instrument médical, notamment pince, ciseaux ou champ comprenant une zone de mâchoires distale constituée par deux mâchoires (2, 12, 22, 34), et un système de manipulation (3, 13, 23, 32) proximal lors de l'actionnement duquel au moins une mâchoire peut être déplacée par rapport l'autre, l'instrument comprenant également un dispositif destiné a régler une force de fermeture maximale, définie, de la zone de mâchoires, caractérisé en ce que sur le parcours de la transmission des forces du système de manipulation (3, 13, 23, 32) à la zone de mâchoires est prévu au moins un élément de transmission de forces (7, 20, 28, 31, 36) en un matériau superélastique à déformation réversible et à mémoire de forme mécanique, dont le diagramme contrainte-allongement traverse, lors de la sollicitation par des contraintes, un palier représentant la zone de superélasticité, en ce que la contrainte de palier (σ 1) est adaptée à la force de fermeture maximale évoquée, et en ce que la course de déplacement du système de manipulation pour fermer la zone de mâchoires est limitée par une butée (8) de manière telle, que l'élément de transmission de forces (7, 20, 28, 31, 36) puisse être sollicité au maximum avec la contrainte de palier.

2. Instrument médical selon la revendication 1, dans lequel les mâchoires (2, 12, 22) sont disposées à l'extrémité distale et le système de manipulation (3, 13, 23) à l'extrémité proximale d'un corps tubulaire (1, 11, 21), le système de manipulation comprenant une pièce de poignée (4, 14, 24) reliée de manière rigide au corps tubulaire et une pièce de poignée mobile (5, 15, 25) associée à la précédente, caractérisé en ce que l'élément de transmission de forces est réalisé sous forme d'une tige de traction et de poussée (7), qui est guidée dans le corps tubulaire (1) et qui relie les mâchoires (2) à la pièce de poignée mobile (5) en ce sens que l'élément de transmission de forces (7) est sollicité par une force de traction pour la fermeture des mâchoires.

3. Instrument médical selon la revendication 1, dans lequel les mâchoires (2, 12, 22) sont disposées à l'extrémité distale et le système de manipulation (3, 13, 23) à l'extrémité proximale d'un corps tubulaire (1, 11, 21), le système de manipulation comprenant une pièce de poignée (4, 14, 24) reliée de manière rigide au corps tubulaire et une pièce de poignée mobile (5, 15, 25) associée à la précédente, caractérisé en ce que l'élément de transmission de forces est réalisé en tant qu'élément de flexion (20), qui est disposé entre la pièce de poignée mobile (15) et l'extrémité proximale d'une tige d'actionnement (17) guidée dans le corps tubulaire (11) et reliant la pièce de poignée mobile (15) aux mâchoires (12).

4. Instrument médical selon la revendication 1, dans lequel les mâchoires (2, 12, 22) sont disposées à l'extrémité distale et le système de manipulation (3, 13, 23) à l'extrémité proximale don corps tubulaire (1, 11, 21), le système de manipulation comprenant une pièce de poignée (4, 14, 24) reliée de manière rigide au corps tubulaire et une pièce de poignée mobile (5, 15, 25) associée à la précédente, caractérisé en ce que l'élément de transmission de forces est réalisé en tant que ressort de traction (31) guidé dans le corps tubulaire (21), et dont l'extrémité proximale est en liaison avec la pièce de poignée mobile (25), et dont l'extrémité distale est en liaison avec les mâchoires (22).

5. Instrument médical selon la revendication 1, dans lequel les mâchoires (2, 12, 22) sont disposées à l'extrémité distale et le système de manipulation (3, 13, 23) à l'extrémité proximale d'un corps tubulaire (1, 11, 21), le système de manipulation comprenant une pièce de poignée (4, 14, 24) reliée de manière rigide au corps tubulaire et une pièce de poignée mobile (5, 15, 25) associée à la précédente, caractérisé en ce que l' élément de transmission de forces est réalisé en tant que ressort de compression (28) guidé dans le corps tubulaire (21), et dont l'extrémité proximale est en liaison avec la pièce de poignée mobile (25), et dont l'extrémité distale est en liaison avec les mâchoires (22).

6. Instrument médical selon la revendication 1, présentant un mode de construction du type ciseau et comprenant une paire d'éléments de poignée (32), qui est dotée, côté proximal, d'un élément de réception de doigts (33), et côté distal, d'une paire de mâchoires (34), les deux éléments de poignée (32) comprenant un palier de pivotement commun, caractérisé en ce qu'en guise d'élément de transmission de forces est prévue une pièce intermédiaire (36) en un alliage à mémoire de forme, qui constitue une partie intégrante d' au moins l'un des éléments de poignée (32).
